# EUROPEAN PATENT APPLICATION

(11) **EP 2 495 011 A1**
(43) Date of publication of application: **05.09.2012**
(21) Application number: 12170063.7
(22) Date of filing: 25.02.2008
(51) Int. Cl.: A61N 1/05, A61B 5/0478, A61N 1/36

(54) **Electrode system for deep brain stimulation**

(30) Priority: 02.03.2007 EP 07103401
(62) Divisional of application: 08719463.5
(71) Applicant: Sapiens Steering Brain Stimulation B.V., 5656 AE Eindhoven (NL)
(72) Inventor: Martens, Hubert C., F., 5656 AE Eindhoven (NL); Decré, Michel M., J., 5656 AE Eindhoven (NL); Cantatore, Eugenio, 5656 AE Eindhoven (NL)
(74) Representative: Rupprecht, Kay

(57) **Abstract**

The invention relates to an electrode system (200) that is particularly suited for deep brain stimulation. According to a preferred embodiment, the electrode system (200) comprises an elongated probe body (202) carrying a plurality of annular stimulation electrodes (201) of radius r and axial extension h that are axially distributed at distances d. The axial extension h is preferably smaller than the diameter 2r and preferably larger than the distance d. Moreover, the electrode system (200) optionally comprises a plurality of microelectrodes (203) projecting radially away from the probe body (202), said microelectrodes (203) being suited for recording neurophysio logic potentials.

## Description

The invention relates to an electrode system for deep brain stimulation comprising an elongated probe body with a plurality of stimulation electrodes. Electrical stimulation of brain regions by implanted electrodes is a possible therapy for several neural disorders. The US 6 343 226 discloses an electrode system for such a deep brain stimulation that comprises a flexible, axially extending probe body with several annular stimulation electrodes distributed at equal distances along a region of the probe body and an axially movable stilette that can be pushed ahead from the tip of the probe body into the tissue and that serves as an electrode for recording physiological potentials. The document does not go into detail with respect to the dimensions of these electrodes.

Based on this background it was an object of the present invention to provide means for improving the therapeutic effect of deep brain stimulation or similar electrophysiological interventions.

This object is achieved by an electrode system according to claim 1 and a method according to claim 10. Preferred embodiments are disclosed in the dependent claims.

According to its first aspect, the invention relates to an electrode system that is particularly suited for deep brain stimulation (i.e. as a "deep brain stimulation system"), though it is also favorably usable in various other applications. The electrode system comprises following components:
a) An axially extending "probe body", i.e. a body with a typically elongated or filamentary shape, wherein the direction of extension of this shape is by definition the "axis" of the probe body. The probe body is typically made from a flexible, physiologically compatible and electrically isolating material, for example from polyimide, or polyurethanes and silicone-urethane copolymers.
b) At least three electrodes that are distributed along the axis of the probe body, wherein these electrodes are also called "stimulation electrodes" in the following for purposes of reference and as an indication of their typical function, i.e. the stimulation of neural tissue. The stimulation electrodes are typically of equal shape and size and disposed in axial direction at equal distances, though the use of differently shaped and/or sized electrodes disposed at different distances from each other shall also be comprised by the invention. Moreover, the stimulation electrodes typically have the shape of a ring or a disk.

The diameter 2r of the stimulation electrodes (with r being the radius of the electrodes) shall be larger than the axial extension h of the electrodes. Written as a formula this is tantamount to saying that the "aspect ratio" h/2r < 1 (it should however be noted that formulas like this shall not imply a sharp boundary for the scope of the claims as e.g. aspect ratios slightly larger than 1 will of course still provide the positive effects of the invention). By definition, the "diameter" of the stimulation electrodes is measured in a direction perpendicular to the axis of the probe body, while the "axial extension" is of course measured in the direction of said axis. If the outline of the electrodes is not circular, the diameter has to be defined appropriately, for example as the maximal possible distance between two points lying on the contour of the electrode.

The number of electrodes is preferably at least as large as 2r/(h+d) or even as 2r/h, with d being the (mean) distance between neighboring electrodes. This guarantees that the electrodes extend over an axial length H that is comparable to the diameter of the probe body.
c) A controller for selectively generating patterns of electrical potentials that differ from each other in that they are shifted in axial direction with respect to the stimulation electrodes. The patterns preferably comprise the application of identical electrical potentials (e.g. 3 V) to a group of n (n = 2; 3; 4; ...) stimulation electrodes, wherein n is smaller than the total number M of stimulation electrodes and wherein the electrodes are preferably neighbors of each other; most preferably, the residual (M-n) stimulation electrodes are clamped to another fixed potential (e.g. 0 V) or floating. A single pulse generator will then suffice in this case to drive the controller.

The controller may optionally be able to selectively address the stimulation electrodes, i.e. apply an individual potential to each stimulation electrode; the volume of activation can then be adjusted within a large range with respect to its position and size.

As will be shown in more detail with respect to the Figures, the proposed aspect ratio h/2r < 1 of the stimulation electrodes is advantageous with respect to the volume of activation that is stimulated in neural tissue by electrical potentials applied to the electrodes. The limited axial height h of the electrodes with respect to their diameter 2r has particularly the effect that the volume of activation is comparatively small and well localized in axial direction. Furthermore, the controller can selectively shift the volume of activation in the surrounding neural tissue along the axial direction of the electrode system in steps of the (small) distance between two stimulation electrodes. Thus it is possible to adapt the electrical stimulation of the electrode system precisely to the brain region where it is needed.

Preferably, the diameter 2r of the stimulation electrodes is at least twice as large as their axial extension, i.e. 2r > 2h, and most preferably it is even four times larger than the axial extension, i.e. 2r > 4h.
In another particular embodiment of the invention, at least two neighboring stimulation electrodes have an axial distance d from each other that is smaller than their axial extension h, i.e. d < h. More preferably an even closer inter-electrode spacing may be used, e.g. d < h/2. Preferably all stimulation electrodes of the electrode system comply with such a condition. If the axial extension h is not the same for all electrodes, the condition refers to the maximal axial extension of the considered two neighboring stimulation electrodes. An advantage of this relatively dense electrode placement is (i) that the electrical stimulation of neural tissue can be very precisely located by shifting the activation pattern from one electrode to the next and (ii) that the electrical impedance of the electrode-tissue system is not too high because of the relatively large electrode surface area when using relatively small inter-electrode spacing.

The stimulation electrodes are preferably distributed over an axial region with a length H that is at least as long as the diameter 2r of the stimulation electrodes, i.e. H > 2r, preferably at least two times as long as said diameter, i.e. H > 2-2r, most preferably at least five times as long as said diameter, i.e. H > 5-2r. Alternatively, said length H is requested to be at least ten times as long as the axial extension h of the electrodes, i.e. H > 10-h. This guarantees that there is a sufficiently long distance over which the stimulation of the electrodes can be distributed and over which the centre of gravity of the stimulation can be adjusted electrically without moving the electrode system physically. In typical cases, H ranges between 1 mm and 20 mm.

The controller preferably comprises a single pulse generator that can generate voltage pulses with a desired (adjustable) frequency and voltage level. By selectively distributing these pulses to the stimulation electrodes, various activation patterns and therefore volumes of activation can be generated. It is a considerable advantage and simplification of the system design that a single pulse generator suffices to create a flexible stimulation volume.

According to a further development of the invention, the electrode system comprises at least one microelectrode projecting away from the probe body, i.e. originating at the surface of the probe body and assuming at least at some point a larger radial distance from the probe body than at its origin. The microelectrode may particularly extend - at least with a component - in radial direction. The term "microelectrode" is used here to distinguish this electrode from the stimulation electrodes. Moreover, the term indicates that this electrode is usually smaller than the stimulation electrodes, which is due to the fact that the stimulation electrodes are used for electrically stimulating regions with a plurality of neurons while the microelectrode is typically used for recording electrical potentials from only a few neurons or even a single neuron. The microelectrode is usually arranged somewhere between a point immediately in front of the axially first and a point immediately beyond the axially last stimulation electrode. Moreover, the microelectrode typically extends some distance away from the probe body (i.e. during an application into the surrounding neural tissue), said distance being preferably in the order of 100 micrometer or more in order to minimize detrimental effects on quality of recorded neural signals by scar tissue that builds up around the probe body during prolonged implantation in neural tissue. The described electrode system with the microelectrode has the advantage that its microelectrode extends right into the neural tissue that is electrically stimulated by the stimulation electrodes, thus allowing a direct observation of the stimulation effects.

In electrode systems that comprise a microelectrode, this microelectrode is preferably surrounded by an electrical isolation everywhere besides at its tip. This guarantees that only the tip of the microelectrode is sensitive for electrophysiological potentials, wherein said tip can be located sufficiently far away from the probe body for avoiding interferences with the electrical potentials of the stimulation electrodes and for minimizing encapsulation during prolonged implantation.

The microelectrode that projects away from the probe body may in general originate everywhere from the lateral surface of the probe body. It may particularly originate between two stimulation electrodes or, alternatively, within the area of a stimulation electrode. In the latter case, the point of origin of the microelectrode is usually encircled by an isolating material, thus safely separating the microelectrode from the corresponding stimulation electrode.

While the above description always included the case that there is only one single microelectrode, the electrode system preferably comprises a plurality of micro electrodes that project away from the probe body in different directions. Electrophysiological potentials can then be sensed in various directions around the elongated electrode system.

In still another embodiment of the invention, the electrode system with a micro electrode comprises a recording unit for sensing electrical potentials via the microelectrode. Thus it is for example possible to monitor the effects of electrical stimulations generated in the neural tissue by the stimulation electrodes.
The invention further relates to a method for the production of an electrode system with a microelectrode of the kind described above, said method comprising the following steps:
a) The prefabrication of a sheet of isolating material with at least one embedded electrical lead, wherein a stripe of the isolating material comprising an end of the lead is cut free by an U-shaped cut in the isolating material.
b) Rolling said sheet around a prefabricated probe body. The aforementioned stripes can then be folded out of the plane of the sheet to project away from the probe body.

These and other aspects of the invention will be apparent from and elucidated with reference to the embodiment(s) described hereinafter. These embodiments will be described by way of example with the help of the accompanying drawings in which:
Figure 1 shows schematically the application of an electrode system according to the present invention for deep brain stimulation;
Figure 2 shows a first embodiment of an electrode system according to the present invention;
Figure 3 illustrates different volumes of neural activation that can be generated with an electrode system like that of Figure 2 by using different numbers and/or positions of active electrodes;
Figure 4 shows an embodiment of an electrode system according to the present invention comprising microwires carrying microelectrodes;
Figure 5 shows an embodiment of an electrode system according to the present invention comprising microstructures carrying microelectrodes;
Figure 6 shows an embodiment of an electrode system according to the present invention comprising microstructures carrying microelectrodes that originate within stimulation electrodes;
Figure 7 illustrates a production method for an electrode system with microelectrodes.

Like reference numbers or numbers differing by integer multiples of 100 refer in the Figures to identical or similar components.

The beneficial therapeutic effects of the application of small electric stimuli to central nervous tissue have been discovered by Benabid and co-workers (Grenoble) in the late 1980s. Applying the so-called high-frequency electrical stimulation (130 Hz, 3 V, 60 µs, typical stimulation parameters) to thalamic structures could relieve both Parkinson's disease (PD) patients and Essential Tremor (ET) patients from their tremor. In later years, other targets for deep brain stimulation (DBS) have been identified (e.g. internal segment of the globus pallidus, GPi, and subthalamic nucleus, STN) that resulted in marked improvements of quality of life of PD patients. Moreover, the use of DBS for other neurological disorders like epilepsy and depression is being examined.

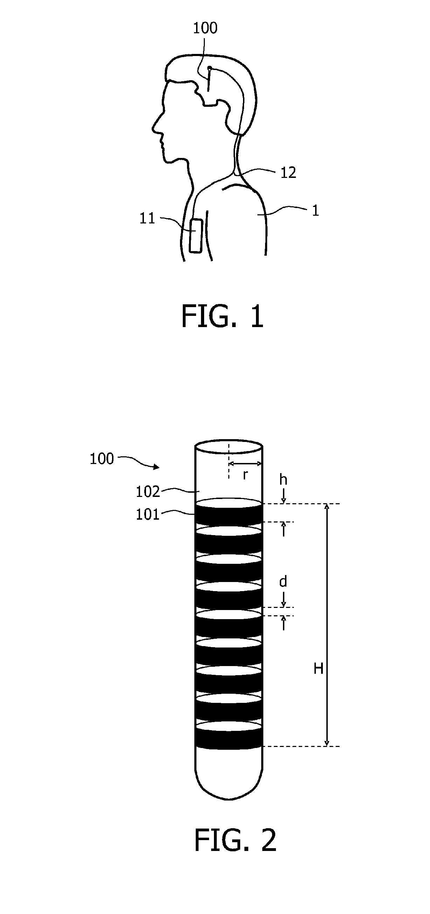

A typical DBS system configuration is shown in Figure 1 and consists of: an implanted pulse generator 11 that is surgically implanted below the clavicle and supplies the necessary voltage pulses, an extension wire 12 connected to the pulse generator 11 and running through the neck to the skull where it terminates in a connector, and - the DBS probe 100 that is implanted in the brain tissue through a burr-hole in the skull.

It is well known in the practice of DBS therapy that a successful clinical outcome is highly dependent on the accurate positioning of the electrode within the target area, e.g. the subthalamic nucleus. To ensure the accurate placement of the chronic stimulation electrodes careful surgery planning and navigation is performed based on pre-operatively acquired imaging data of the target area in the patient's brain. Subsequently, prior to the implantation of the chronic stimulation electrode, during DBS surgery the medical team performs an electrophysiological exploration of the target area using recording micro-electrodes and subsequently uses acute test stimulation to investigate the effect of stimulation on disease symptoms. These procedures are performed to more closely define the optimum position for chronic stimulation.

Despite the careful surgical, neurophysiological, and neurological procedures it is unavoidable that the chronic stimulation electrode is usually not positioned optimally for DBS therapy. Positional uncertainty may for example arise from inaccuracy of pre-operative imaging data, mechanical imprecision of the targeting system, mechanical disturbance during the probe fixation, and mechanical shifts of brain tissue during surgical and/or implantation procedures.

Another issue is related to the fact that on a patient-to-patient basis there are variations in the detailed anatomical morphology. The precise locations as well as the sizes and shapes of brain structures (including DBS targets like STN or GPi) are not completely identical amongst different individuals. Consequently, the required optimum stimulation field layout differs somewhat from patient-to-patient and in general the optimum shape of stimulation fields is not known a-priori.

Flexibility is therefore needed in the shaping of the stimulation fields in order to correct post-operatively for uncertainty/error of the probe position with respect to the ideal target and in order to cope with uncertainty in the stimulation field requirements based on patients' local detailed anatomical morphology.
With respect to the size of anatomical targets (few mm) and the required accuracy of stimulation field placement (< 1 mm), the typical DBS probes that are being used today for chronic stimulation are too coarse to adapt the stimulation fields to this accuracy.

A known solution to refine the stimulation field positioning is by means of electrical field steering, see e.g. US 589 416. In this case one balances the applied currents (or potentials) to the electrodes in order to shift the stimulation field along the probe direction. Unfortunately, this method has several disadvantages. First of all, the electronic implementation is more difficult since each electrode requires a separate stimulator to address it. Secondly, the shifting of the position of the volume of neuronal activation requires very precise control over the current amplitudes. Thirdly the shifting of the position of the volume of neuronal activation is accompanied by a large change of its shape: the volume of activation does not really shift smoothly along the probe. Instead it "sticks" to the electrode positions resulting in pear- shapes activation volumes even for very refined current-redistributions of 29/30 vs 1/30. The width of such "pear-shaped" volume is determined by the ratio of current amplitudes at the respective electrodes. From the device-design point-of-view this approach is unwanted since the more complicated electronics needed for field-steering methods hampers device miniaturization and increases device cost. From the clinical point-of-view the method is sub-optimal because of the large changes in shape of the volume (it becomes more elongated along probe direction) of neuronal activation when attempting to move its position along the probe.

In the following, various embodiments of electrode systems will be proposed that address the above problems.

Figure 2 shows a first embodiment of a "DBS probe" or "electrode system" 100 that can be applied in the setup of Figure 1. The electrode system 100 comprises: an elongated or filamentary, flexible probe body 102 consisting of an isolating material and having a cylindrical shape with radius r;
a set of stimulation electrodes 101 which appear as rings with an axial extension h and a diameter 2-r on the lateral surface of the probe body 102.

The stimulation electrodes 101 are spaced apart from each other by a distance d, and the whole region of the probe body 102 that is covered by stimulation electrodes 101 extends axially over a length H. While the axial extension h of the stimulation electrodes 101 and the distance d between them may in principle be different for each electrode or pair of electrodes, respectively, Figure 2 shows the preferred case that all axial extensions h and distances d are the same.

A central aspect of the described design of the DBS probe 100 is the refined distribution of electrodes 101 along the probe's axis. Thus the electrodes 101 are characterized by an aspect ratio between axial extension h and diameter 2r that is smaller or equal to 1, h/2r < 1, more preferably this aspect ratio is h/2r < 0.5. In specific embodiments, h/2r < 0.25 may even be chosen. The distance d between electrodes is set preferably to a value that is equal or smaller than the axial extension, d/h < 1, more preferably d/h < 0.5. With such a design, the shape and position of the volume of neuronal activation (VOA) can be controlled to a high degree of accuracy by connecting multiple electrodes in parallel to the output of just a single pulse-generator. This allows shifting of the VOA along the axis, as well as to elongate or compress the VOA along the direction of the probe axis.

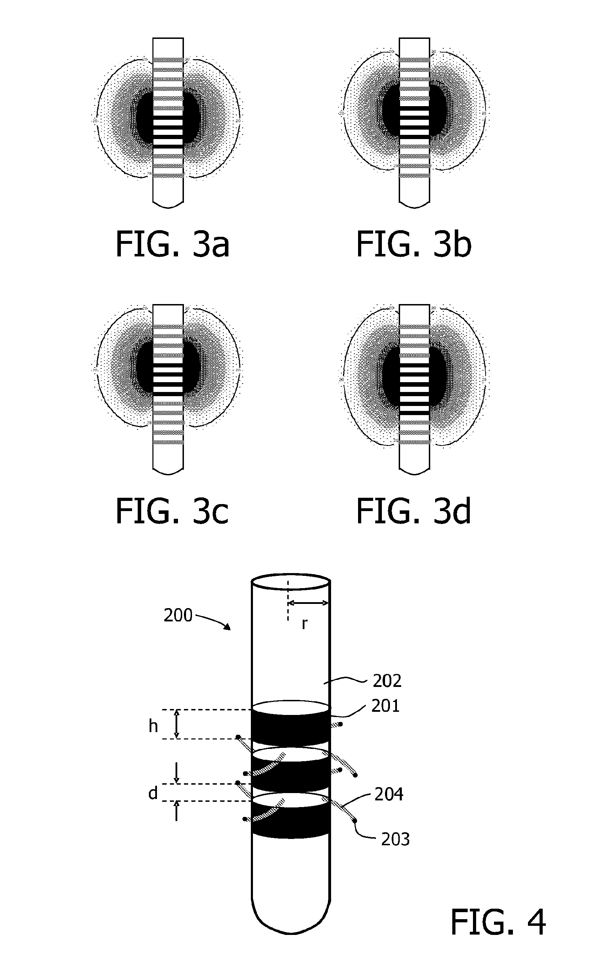

Figure 3 illustrates this with the help of deep brain stimulation computational models. The diagrams show the spatial distribution of the so-called activating function AF for fibers passing the DBS probe in a plane oriented radially with respect to the probe (so-called tangential fibers). The Figure shows the distribution of AF for monopolar stimulation through several neighboring electrodes (indicated in solid black) of a DBS probe like that of Figure 2 carrying 13 electrodes with r = 0.6 mm, h/2r = 0.166; h/d = 1. The drawn lines indicate the boundary where AF = + 20 mV which is a typical value for the excitation of neuronal fibers. Stimulation is set at -3.6 V amplitude. The particular settings of the different diagrams are as follows:
(a) -3.6 V applied at electrodes 4 to 7;
(b) -3.6 V applied at electrodes 5 to 8;
(c) -3.6 V applied at electrodes 6 to 9;
(d) -3.6 V applied at electrodes 4 to 9.

Diagrams (a), (b), (c) show that the stimulation field distribution can be shifted along the probe in a gradual fashion by stepping between subsequent groups of electrodes, while diagram (d) shows that the shape of the activation volume can be adjusted smoothly by changing the number of activated electrodes. Further simulation data on segmented electrode systems can be found in literature (e.g. Xuefeng F Wei and Warren M Grill, "Current density distributions, field distributions and impedance analysis of segmented deep brain stimulation electrodes, J. Neural Eng. 2 (2005) 139-147).

Figures 4 to 7 show different embodiments of electrode systems according to the present invention that comprise, additionally to the embodiment 100 of Figure 2, a plurality of microelectrodes projecting radially away from the probe body. These designs are proposed in view of the following background: DBS electrodes that are being used today contain only macroscopic stimulation electrodes (mm size) and do not allow the recording of the signals (action-potentials) of neurons. To record such neural signals, so-called micro-electrodes (< 100 µm size) are needed that can pick up the small extra-cellular potentials generated by the neurons. The reason for resorting to micro-electrodes for picking up the neural signals is related to the small amplitude of the signals as well as to the typical packing density of neurons. Typically, the size of neuronal cells falls in the range 30 - 50 µm. If the recording electrode is much larger in size, it will average out the firing of multiple neurons and it becomes impossible to discern the individual firing patterns. Also because of the small signal amplitudes, the electrode ideally speaking should be positioned very close to the neuron, which is only possible for electrode sizes that are of same size as the neurons them self. These signal amplitudes can be estimated as follows. The typical membrane currents I during action potential propagation is related to the membrane capacitance C of a cell (10 pF) and the action potential's amplitude U (0.1 V) and duration (0.1 ms) as follows: I = C-(dU/dt) = 10" ^{π}-0.1/10^{∼4} A = 10 nA. The resulting extracellular potential can be estimated by a point-source approximation and yields: U(r) = I/(4πrσ) = 2.5 µV at r = 1 mm distance and 100 µV at the typical inter-neuronal distance of 40 µm.

During DBS surgery, prior to implantation of the chronic stimulation electrodes, such micro-electrode recordings can be used to identify the electrophysiological hall-mark signals of the stimulation targets. Moreover, it would be advantageous in the field of DBS to have the possibility of long-term (chronic) recording of neural signals, such as action potentials, as this would allow studying the evolution of neural signals over prolonged periods of stimulation and might even open possibilities for "closed- loop" stimulation whereby the stimulation output is coupled to recorded neural firing patterns. A problem occurring in this respect is however that, over the course of time, chronically implanted probes carrying recording micro-electrodes lose their ability to pick up neuronal signals. Existing micro-electrode probes are therefore not suitable for long-term DBS applications that need to function for tens of years. A reason for this fact is that tissue response near a probe results in an encapsulation of the probe with a sheath of scar tissue that is approximately 100 µm thick and that is characterized by severely reduced neuronal cell density and enhanced density of microglia. This problem is especially well known from the field of micro-electrode cortical prostheses and it is even more severe around chronically implanted DBS probes that have mm-dimension and that result in large mechanical displacement of tissue. The consequence of this encapsulation sheath is that the micro-electrodes lose "physical" contact with nearby neurons and the neural signals (amplitude drops below 10 µV range) disappear in the noise.

The solution proposed here is to fabricate the micro-electrodes on micro-wire extensions that sprout out of the macroscopic DBS probe. Since tissue response is driven by processes at the cellular level, feature sizes that are smaller than, or of the same magnitude, as cellular features, the resulting cellular responses are much milder, i.e. small devices or processes result in much less severe tissue reactivity. The reduced tissue reactivity at the micro-electrode locations improves the electrical contact and allows for long-term neuronal recording in DBS applications or any other neurostimulation device.

A first specific embodiment of the described solution is shown in Figure 4. Similar to the probe 100 of Figure 2, this electrode system 200 comprises a cylindrical DBS probe body 202 of typically 2r = 1 mm diameter, having four ring-shaped macroscopic stimulation electrodes 201 of h = 1 mm height distributed along the length of the probe with d = 0.5 mm spacing. In each of the three inter-electrode areas, four micro-structured processes 204 extending from the probe surface are distributed at regular intervals along the probe's circumference. The processes have typically a diameter of about 80 µm and a length of about 120 µm. At the distal portion of these processes a recording micro-electrode 203 (20 µm diameter) is located. The conductive portions of the recording micro-electrode are preferably fabricated from biocompatible metals like Pt, Ir, Pt-Ir alloy, or W. Moreover, a coating may be applied on the surface of the micro-electrode that is exposed to the tissue. Such coatings, based e.g. on hydrogel or (conducting) polymer, are used to improve tissue-electrode contact. Though the micro-electrodes 203 are shown to extend primarily in radial direction, they might alternatively also have at least partially a tangential or even recurrent extension.

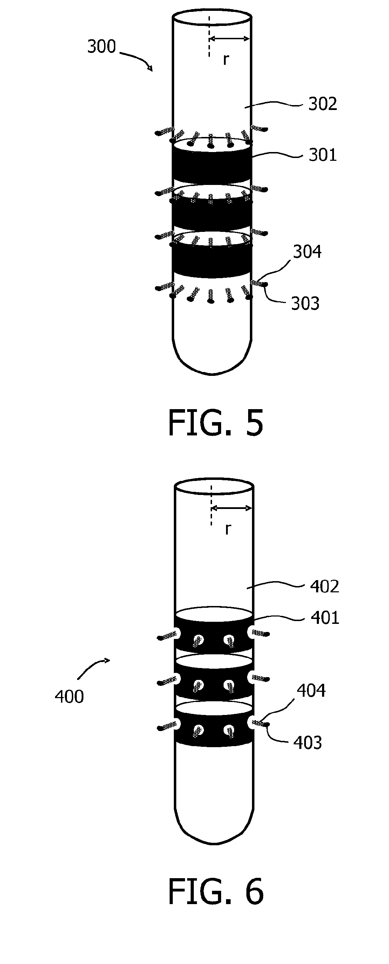

Figure 5 shows a second embodiment of an electrode system 300 according to the invention. In this design microstructures 304 carrying micro-electrodes 303 extend from the surface of the DBS probe 302 originating from the annular spaces between or next to the annular stimulation electrodes 301. The microstructures 304 are somewhat shorter and they are arranged more densely in comparison to the processes 204 of Figure 4. Besides this, their design may be similar or identical.

A third embodiment of an electrode system 400 is shown in Figure 6. This electrode system 400 differs from that of Figure 5 in that the microstructures 404 carrying micro-electrodes 403 extend from the surface of the DBS probe 402 from regions within the stimulation electrodes 401, i.e. they are embedded in the stimulation electrodes.

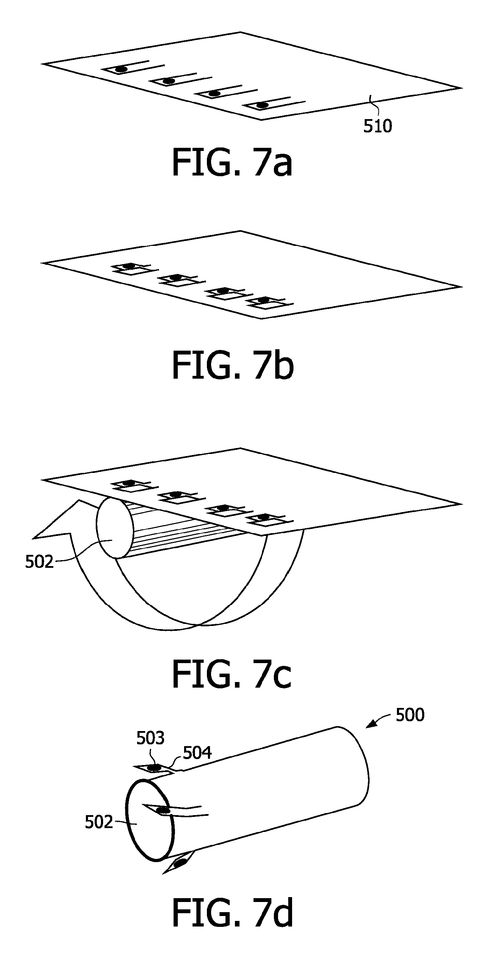

Figure 7 illustrates consecutive steps of an exemplary fabrication procedure for a DBS probe 500 with micro-electrodes 503 on micro-extensions 504. The procedure starts at step (a) with a sheet 510 of isolating material comprising a plurality of parallel running, embedded electrical leads. A stripe of this isolating material comprising a free end of the leads is cut free by an U-shaped cut.

In the next step (b), the cut-free ends of the isolating material are bent upwards out of the plane of the sheet. In step (c), the sheet is rolled around and attached to a cylindrical probe body 502 consisting for example of polyimide. This results in the final electrode system 500 with micro-extensions 504 projecting radially from the probe body and carrying free micro-electrodes 503 at their distal ends.

In summary, a novel deep-brain-stimulation probe design with refined distribution of electrodes along the probe's axis was proposed. According to one aspect of this proposal, the stimulation electrodes are characterized by an aspect ratio h/2r < 1 , more preferably h/2r < 0.5, and in some instances even h/2r < 0.25, while the aspect ratio is typically limited at the lower side of the spectrum by h/2r > 0.05 and more preferably h/r > 0.10. The distance d between electrodes is preferably d/h < 1 and more preferably d/h < 0.5. The new probe design allows a refined shaping and positioning of the volume of neuronal activation around the probe by connecting appropriate groups of electrodes to the stimulator output. In another aspect of the invention, a probe design was proposed comprising microelectrodes extending away from the probe body that carries the stimulation electrodes.

Finally it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

In particular, the present invention relates to the following aspects, which are hereinafter explicitly disclosed:
1. An electrode system (100-500) for deep brain stimulation, comprising
   a) an axially extending probe body (102-502);
   b) at least three stimulation electrodes (101-501) that are distributed along the axis of the probe body (102-502), wherein the diameter 2r of the stimulation electrodes (101-501) is equal or larger than their axial extension h: 2r > h;
   c) a controller (11) for selectively generating patterns of electrical potentials that differ from each other in that they are shifted in axial direction with respect to the stimulation electrodes.
2. The electrode system (100-500) according to aspect 1,
   **characterized in that** the diameter 2r of the stimulation electrodes (101-501) is at least twice as large as their axial extension, 2r > 2h, preferably at least four times larger than their axial extension, 2r > 4h.
3. The electrode system (100-500) according to aspect 1,
   **characterized in that** at least two neighboring stimulation electrodes (101-501) have a distance d that is smaller than the axial extension h of the electrodes according to d < h, preferably to d < 0.5-h.
4. The electrode system (100-500) according to aspect 1,
   **characterized in that** the stimulation electrodes (101-501) are distributed over an axial region with a length H that is at least as long as the diameter 2r of the stimulation electrodes (101-501) and/or that is at least ten times as long as the axial extension h of the electrodes: H > 10-h.
5. The electrode system (100-500) according to aspect 1,
   **characterized in that** the controller (11) comprises a single pulse generator.
6. The electrode system (200-500) according to aspect 1,
   **characterized in that** it comprises at least one microelectrode (203-503) projecting away from the probe body (202-502).
7. The electrode system (200-500) according to aspect 6,
   **characterized in that** the microelectrode (203-503) is surrounded by an electrical isolation (204-504) everywhere besides at its tip.
8. The electrode system (200-500) according to aspect 6,
   **characterized in that** the microelectrode (203-503) originates between two stimulation electrodes (201, 301) or within the area of a stimulation electrode (301).
9. The electrode system (100-500) according to aspect 6,
   **characterized in that** it comprises a recording unit (11) for sensing electrical potentials via the microelectrode (203-503).
10. A method for the production of an electrode system (200-500) according to aspect 6, comprising
   a) the fabrication of a sheet (510) of isolating material with at least one embedded electrical lead, wherein a stripe of the isolating material comprising an end of the lead is cut free;
   b) rolling the sheet (510) around a probe body (502).

## Claims

1. An electrode system (100-500) for deep brain stimulation, comprising
a) an axially extending probe body (102-502);
b) at least three stimulation electrodes (101-501) that are distributed along the axis of the probe body (102-502), wherein the diameter 2r of the stimulation electrodes (101-501) is equal or larger than their axial extension h: 2r ≥ h;
c) a controller (11) for selectively generating patterns of electrical potentials that differ from each other in that they are shifted in axial direction with respect to the stimulation electrodes,
whereby the electrode system is configured such that its stimulation field distribution can be shifted along the probe in a gradual fashion by stepping between subsequent groups of electrodes and whereby the shape of the activation volume can be adjusted smoothly by changing the number of activated electrodes.

2. The electrode system (100-500) according to claim 1,
**characterized in that** the diameter 2r of the stimulation electrodes (101-501) is at least twice as large as their axial extension, 2r > 2h, preferably at least four times larger than their axial extension, 2r > 4h.

3. The electrode system (100-500) according to claim 1,
**characterized in that** at least two neighboring stimulation electrodes (101-501) have a distance d that is smaller than the axial extension h of the electrodes according to d < h, preferably to d < 0.5-h.

4. The electrode system (100-500) according to claim 1,
**characterized in that** the stimulation electrodes (101-501) are distributed over an axial region with a length H that is at least as long as the diameter 2r of the stimulation electrodes (101-501) and/or that is at least ten times as long as the axial extension h of the electrodes: H > 10-h.

5. The electrode system (100-500) according to claim 1,
**characterized in that** the controller (11) comprises a single pulse generator.

6. The electrode system (200-500) according to claim 1,
**characterized in that** it comprises at least one microelectrode (203-503) projecting away from the probe body (202-502).

7. The electrode system (200-500) according to claim 6,
**characterized in that** the microelectrode (203-503) is surrounded by an electrical isolation (204-504) everywhere besides at its tip.

8. The electrode system (200-500) according to claim 6,
**characterized in that** the microelectrode (203-503) originates between two stimulation electrodes (201, 301) or within the area of a stimulation electrode (301).

9. The electrode system (100-500) according to claim 6,
**characterized in that** it comprises a recording unit (11) for sensing electrical potentials via the microelectrode (203-503).

10. A method for the production of an electrode system (200-500) according to claim 6, comprising
a) the fabrication of a sheet (510) of isolating material with at least one embedded electrical lead, wherein a stripe of the isolating material comprising an end of the lead is cut free;
b) rolling the sheet (510) around a probe body (502).
